(19) Europäisches Patentamt

European Patent Office

Office européen des brevets

(11) **EP 1 479 731 A1**

(12) **EUROPEAN PATENT APPLICATION**

| | |
|---|---|
| (43) Date of publication:<br>**24.11.2004 Bulletin 2004/48** | (51) Int Cl.$^7$: **C09C 1/00** |

(21) Application number: **04009622.4**

(22) Date of filing: **23.04.2004**

| | |
|---|---|
| (84) Designated Contracting States:<br>**AT BE BG CH CY CZ DE DK EE ES FI FR GB GR**<br>**HU IE IT LI LU MC NL PL PT RO SE SI SK TR**<br>Designated Extension States:<br>**AL HR LT LV MK** | (72) Inventors:<br>• **Heider, Lilia, Dr.**<br>  **Winchester SO22 4HZ (GB)**<br>• **Riddle, Rodney, Dr.**<br>  **Poole Dorset BH12 3AG (GB)**<br>• **Weller, Mark T., Prof.**<br>  **Old Basing Basingstoke RG248WE Hants (GB)**<br>• **Longhurst, Rayne W., Dr.**<br>  **Salisbury SP13SB Wiltshire (GB)** |
| (30) Priority: **20.05.2003 EP 03011415** | |
| (71) Applicant: **Merck Patent GmbH**<br>**64293 Darmstadt (DE)** | |

(54) **Coloured pigments comprising complex phosphate systems**

(57)    The present invention relates to a novel effect pigment based on substrates comprising at least one layer, which consists of a coloured complex phosphate system, the production of these pigments and their use.

EP 1 479 731 A1

## Description

The present invention relates to a novel effect pigment based on substrates comprising at least one layer, which consists of a coloured complex phosphate system, the production of these pigments and their use.

Existing lustre effect pigments are based on metal oxide coatings on suitable substrates. This can include multiple layers of different oxides on various substrates including mica, silicon dioxide or aluminium oxide. These products have colours generated by interference effects and absorption properties of the metal oxides combined with a lustrous appearance. However, without addition of absorbing materials, such as inorganic absorbing metal oxides, carbon black or organic dyes, a limited range and depth of colours only is possible in these pigments.

JP 21456/1983 describes colour flake pigments based on non-metallic inorganic flake pigments coated with an outer layer of an insoluble metal phosphate as a binding vehicle containing an insoluble organic pigment. The metal of the insoluble metal phosphate can be selected from magnesium, calcium, zinc, aluminium, titanium or zirconium. Due to the transparency of the above-mentioned metal phosphates the absorption colour of the outer layer is based on the organic pigment. The coprecipitation of the organic pigment into a metal phosphate layer is disadvantageous in respect of a possible non-uniform distribution of the absorption pigment within the metal phosphate.

It is therefore an object of the present invention to provide new coloured effect pigments, which combine a lustre or brilliant appearance with a great colour intensity and hue. Furthermore, the effect pigments should advantageously show a uniform colour appearance.

Surprisingly it has been found that effect pigments according to the present invention can fulfil these objectives in a more effective manner. Therefore, the present invention describes effect pigments based on substrates comprising at least one layer, which consists of a coloured complex phosphate system.

Such objects are achieved by a process for preparing the pigment according to the present invention, in which a substrate is coated with at least one inorganic phosphate and in the case of coating with one inorganic phosphate with at least one further metal component, followed by annealing to form the complex phosphate system.

The invention additionally provides for the use of the pigments of the invention for pigmenting paints, lacquers, printing inks, powder coatings, paper coatings, plastics, cosmetics, inks, glazes for ceramics and glasses, decorative applications for foods and drugs and security-enhancing features. For this purpose they can also be employed as mixtures with commercially customary pigments, examples being organic and inorganic absorption pigments, effect pigments and LCP pigments, e.g. hiding white, coloured and black organic and inorganic pigments and also with conventional transparent, coloured and black lustre pigments based on metal oxide coated mica, $TiO_2$ flakes, $SiO_2$ flakes or $Al_2O_3$ flakes and coated or uncoated metal pigments, $BiOCl$ pigments, platelet-shaped iron oxides or graphite flakes. The inventive pigments can be further coated with organic or inorganic layers to yield combination pigments.

Substrates that can be used in the present invention as base material on which the complex phosphate system is deposited include platelet-shaped, spherical or needle-shaped substrates. Preferably the substrates comprise but are not limited to micaceous iron oxide, natural (for example as in WO 99/48634), synthetic or doped (for example as in EP-A 0 068 311) micas (muscovite, phlogopite, fluoro-phlogopite, synthetic fluorophlogopite, talc, kaolin), basic lead carbonate, platelet-shaped barium sulphate, $SiO_2$-, $Al_2O_3$-, $TiO_2$-, Glass-, ZnO-, $ZrO_2$-, $SnO_2$-, $BiOCl$-, chromium oxide-, BN-, MgO-flakes, magnesium fluoride, $Si_3N_4$, graphite, effect pigments (for example EP-A 9 739 066, EP-A 0 948 571, WO 99/61529, EP-A 1 028 146, EP-A 0 763 573, US-A 5,858,078, WO 98/53012, WO 97/43348, US-A 6,165,260, DE-A 15 19 116, WO 97/46624, EP-A 0 509 352), effect multilayer pigments (for example EP-A 0 948 572, EP-A 0 882 099, US-A 5,958,125, US-A 6,139,613) and/or metals. Preferably, the metal is aluminium and/or titanium, most preferably passivated by inorganic treatment. Furthermore, coated $SiO_2$ spheres (for example EP-A 0 803 550, EP-A 1 063 265, JP-A 11 322 324), uncoated $SiO_2$ spheres (Ronaspheres® , all spheres described as starting materials in EP-A 0 803 550, EP-A 1 063 265, JP-A 11 322 324), micro bubbles (US-A 4,985,380), as well as needle-shaped metal oxides, preferably iron oxide, can be used as substrates to form a non-lustrous coloured pigment. In particular the substrate is of metal oxide coated mica, $Al_2O_3$, $SiO_2$, $TiO_2$ or glass. Especially preferred are multilayer pigments comprising alternating layers of metal oxide, metal oxide hydrate, metal and/or metal fluoride with a refractive index n > 1.8 and a metal oxide, metal oxide hydrate, metal and/or metal fluoride with a refractive index n ≤ 1.8. These multilayer effect pigments are characterised through an intensively lustrous appearance and an angle-dependent interference colour.

The size of the substrates is not critical. Preferably, the mean diameter in the case of for example the platelet-shaped substrates can vary between 5 and 200 μm, preferably between 10 and 150 μm. The mean diameter of the spherical substrates can vary between 10 nm and 100 μm, preferably between 500 nm and 50 μm and most preferably from 1 to 20 μm.

Effect pigments according to the present invention comprise at least one layer of a complex phosphate system. In particular, the complex phosphate system has the formula

$$A_pB_qP_xO_y$$

with

A = ammonium, metals of group 1, 2, 3, 11 and/or 12 of the periodic table and/or lanthanides

B = elements of group 3 to 15 of the periodic table, lanthanides and/or actinides

x = 1,2....n
y = 2,....m.
p = 1 , 2 ....r and
q = 1 , 2 ....s

A and B should enable to fulfil the stochiometric formula, that means that depending on the oxidation state of A and B the final formula can differ.

Preferably, the complex phosphate system may consist of $KNiPO_4$, $CuZr_4P_6O_{24}$, $CuZr_2(PO_4)_3$, $SrMn_2(PO_4)_2$, $(NH_4)MnP_2O_7$, $CsCoPO_4$, $SrCo_2(PO_4)_2$, $BiCu_2PO_6$, $BiMg_2PO_6$, $Cu_2Mg_3(PO_4)_2$, $CaMnP_2O_7$, $Sr_2Cu(PO_4)_2$, $SrFe_3(PO_4)_3O$, $SrCo_3(P_2O_7)_2$, $Ca_9Fe(PO_4)_7$, $SrFe_3(P_2O_7)_2$, $CaZr_2(PO_4)_2$.

The thickness of the layer of the complex phosphate system can vary between 10 and 300 nm, preferably between 20 and 200 nm depending on the required colour effect of the pigments.

For the preparation of the complex phosphate system a substrate as described above is coated with at least one inorganic phosphate and in the case of coating with only one inorganic phosphate with at least one further metal component, followed by annealing to form the complex phosphate system.

The deposition of the inorganic phosphate and/or metal component is performed wet chemically using appropriate precursors. According to the preferred composition under formula $A_pB_qP_xO_y$ of the complex phosphate system, the constituents A and B can be deposited separately as phosphate or metal component onto the substrate prior annealing. The order of the deposition of the phosphate, respectively the metal component, has no influence on the formation of the complex phosphate system afterwards.

The inorganic phosphate can be deposited directly by precipitation using a solution of one constituent of the complex phosphate system and a phosphate source. The phosphate source can be any inorganic phosphate, such as primary, secondary or tertiary alkaline or earth alkaline metal phosphates, e.g. sodium phosphate, Disodiumhydrogenphosphate, Sodium dihydrogenphosphate, Ammonium dihydrogenphosphate, Diammonium hydrogenphosphate, Pyrophosphates, Polyphosphates or Phosphoric acid. Especially preferred are Disodiumhydrogenphosphate, Diammonium hydrogenphosphate, Pyrophosphates, Polyphosphates and Phosphoric acid. Process parameters such as pH-value or temperature of the solution are controlled to obtain optimal conditions for precipitation.

For the precipitation of the constituents of the complex phosphate system in the deposition process all known soluble compounds of these constituents may be used as source, such as halogenides, nitrates, oxalates or other organic acid salts and/or sulphates, preferably the chlorides and/or nitrates. As well as in the case of the phosphates the deposition parameter can be optimised.

In the case that all constituents of the complex phosphate system are deposited in the form of phosphates, the precipitation of a further metal component is not necessary. If only one constituent of the complex phosphate system is deposited onto the substrate in the form of an inorganic phosphate, additionally at least one further metal component has to be precipitated onto the substrate either before or after the phosphate deposition. The metal component can be a hydroxide, oxide hydrate and/or oxyhydrate.

The following embodiments for the preparation of complex phosphate systems with Metal 1 and Metal 2 corresponding to A and B in the formula $A_pB_qP_xO_y$ are preferred:

| Educt | Process Step 1 | Process Step 2 |
|---|---|---|
| Substrate | Metal 1 phosphate layer | Metal 2 layer oxyhydrate/hydroxide layer |
| Substrate | Metal 1 phosphate layer | Metal 2 phosphate layer |
| Substrate Substrate | Simultaneous deposition of Metal 1 phosphate and Metal 2 phosphate layer | |
| Substrate | Metal 1 layer oxyhydrate/hydroxide layer | Metal 2 phosphate layer |

After deposition the resulting product can be filtrated and dried prior annealing. The drying can be performed at temperatures in the range of 80 and 120°C, preferably between 100 and 120°C. The period of drying can vary between 6 and 24 hours, preferably between 8 and 14 hours. Any person skilled in the art can perform the control and optimization of the process parameters.

To transform the above described deposited layer into the complex phosphate system an annealing is performed. The temperature for annealing can vary between 500 and 900°C, preferably between 650 and 850°C. Annealing can be performed under oxidising, reducing and/or inert atmospheres, depending on the desired complex phosphate system. Oxidising atmospheres contain oxygen, air and/or mixtures thereof.

Hydrogen alone or in mixture with nitrogen and/or argon may be used as reducing atmospheres. Pure nitrogen, pure argon and/or mixtures thereof are suited for usage as inert atmospheres. Any person skilled in the art can perform the control and optimization of the process parameters.

The new coloured effect pigments can be used as substrate to precipitate further optical layers. If desired, the pigments according to the present invention can be further coated with one or more layers of metal oxides, metal oxide hydrates, metal fluorides and/or semitransparent metal layers on top of the complex phosphate system. The complex phosphate layer can also be placed as an intermediate layer of metal oxide, metal oxide hydrate, metal fluoride and/or semitransparent metal stacks. The metal oxide can be selected from any metal oxide, preferably from titanium oxide, iron oxide, aluminium oxide, aluminium oxide hydrate, silicon oxide, silicon oxide hydrate, zirconium oxide, chromium oxide, zinc oxide, tin oxide, antimony oxide, indium oxide, potassium ferric ferro cyanides, most preferably from titanium oxide, iron oxide, aluminium oxide, aluminium oxide hydrate, silicon oxide, silicon oxide hydrate and/or mixtures thereof. The metal fluoride is preferably magnesium fluoride. The metal of the semitransparent metal layer can be selected from chromium, molybdenum, aluminium, silver, platinum, nickel, copper and/or gold, preferably from aluminium, silver. In particular, the metal oxide, metal oxide hydrate, metal fluoride and/or semitransparent metal layers are arranged as alternating layers of metal oxide, metal oxide hydrate, metal and/or metal fluoride with a refractive index $n > 1.8$ and a metal oxide, metal oxide hydrate, metal and/or metal fluoride with a refractive index $n \leq 1.8$. Pigments according to this embodiment combine the colour of the complex phosphate system with an intensively lustrous appearance and may show an angle-dependent interference colour.

Preferred examples for metal oxides, metal oxide hydrates and/or metals with a refractive index $n > 1.8$ are titanium oxide, iron oxide, iron titanate, iron, chromium, silver and/or nickel, preferably titanium oxide, iron oxide, iron titanate.

Preferred examples for metal oxides, metal oxide hydrates, metals and/or metal fluorides with a refractive index $n \leq 1.8$ are silicon oxide, silicon oxide hydrate, aluminium oxide, aluminium oxide hydrate, aluminium and/or magnesium fluoride.

Examples for the constitution of pigments according to the present invention are given in the following:

$$\text{Substrate} + BiPO_4 + Cu(OH)_2$$

$$\rightarrow \text{substrate plus complex phosphate system}$$

$$\text{Substrate} + BiPO_4 + Cu_3(PO_4)_2$$

$$\rightarrow \text{substrate plus complex phosphate system}$$

$$\text{Substrate} + TiO_2 + BiPO_4 + Cu(OH)_2$$

$$\rightarrow \text{metal oxide coated substrate plus complex phosphate system}$$

$$\text{Substrate} + TiO_2 + SiO_2 + TiO_2 + BiPO_4 + Cu(OH)_2$$

$$\rightarrow \text{multilayer coated substrate plus complex phosphate system}$$

$$\text{Substrate} + BiPO_4 + Cu(OH)_2 + TiO_2$$

$$\rightarrow \text{complex phosphate system coated substrate plus metal oxide}$$

Substrate + $BiPO_4$ + $Cu(OH)_2$ + $TiO_2$ + $SiO_2$ + $TiO_2$

$\rightarrow$complex phosphate system coated substrate plus multilayer system

Furthermore, the resulting pigments can be coated with inorganic and/or organic compounds to increase their weather stability respectively their photo stability. Useful methods are for instance described in US-A 4,134,776, EP-A 0 649 886, WO 97/29059 and references cited therein.

Pigments according to the present invention show a high value of chroma, good tinting strength, and pure colour and in the case of platelet-shaped substrates an interesting lustre. They offer the generation of new colours that cannot be produced by using systems of the state of the art like $TiO_2$, iron oxides or organic pigments coated substrates. Furthermore, these effects cannot be achieved through physical mixtures of interference colour pigments and absorbing systems. Depending on the type and thickness of the substrate as well as on the hue and colour intensity of the complex phosphate system many different optical effects are possible. Transparent platelet-shaped substrates such as mica offer the creation of lustrous coloured pigments. Coatings on metallic, semi-transparent or coloured substrates provide overlapping colour effects of absorption, reflection and interference combined with a greater hiding strength. In the case of spherical substrates pigments with a great variety in colour are obtainable. Due to their optical behaviour interesting absorption colours without lustre can be observed. In addition and in contrast to pigments comprising organic pigment containing layers, the new effect pigments show a uniform colour.

Furthermore, pigments according to the present invention show a good dispersibility in all application media. The combination of new colour effects combined with good application behaviour in pigments according to the present invention is useful in a wide range of applications, such as paints, lacquers, printing inks, powder coatings, paper coatings, plastics, cosmetics, inks, glazes for ceramics and glasses, decorative applications for foods and drugs and security-enhancing features.

The pigments and their production process according to the present invention is more illustratively demonstrated but not limited by means of the following examples.

**Examples:**

Example 1: Realisation of ca. 50% $BiCu_2PO_6$ onto mica

100 g of mica is suspended in 1.9 litres of fully deionised water. The suspension is heated to 75°C. The pH of the solution is adjusted to pH 1-5, preferable at 4 by addition of nitric acid or hydrochloric acid. Roughly 193 g of a bismuth nitrate solution (12.4% Bismuth content) are slowly added to the reactor. The pH-value is maintained via a relevant amount of Di-sodium hydrogen-phosphate solution (4.8% Phosphate content) to form $BiPO_4$. The suspension is equilibrated for 30 min. Then the pH-value is adapted to 5-9 by dosage of a sodium hydroxide solution (32% NaOH). 112.2 g of a copper chloride solution (15% Cu content) are added to precipitate $Cu(OH)_2$. The pH is maintained using sodium hydroxide (32%).

The suspension is cooled down and the solid remaining is decanted and washed to remove all side products. The pigment is transferred to a porcelain crucible and dried over night at 110°C. After sieving with a 0.3 mm sieve the powder is calcined at 750°C for 30 min. A green $BiCu_2PO_6$ pigment with lustre is obtained:
L value 56, a = -17, b = 32
These values are measured using a Minolta colour unit CR-300. The Lab values can differ depending on the applied layer thickness and morphology.

Example 2:

A green $BiCu_2PO_6$ pigment with lustre according to example 1 can also be obtained by precipitation of Bi $(OH)_3$ followed by precipitation $Cu_3(PO_4)_2$ and subsequent treatment described in example 1.

Example 3: Realisation of 48.6% $SrCo_2(PO_4)_2$ onto mica

100 g of mica are suspended in 1.9 litres of fully deionised water. The suspension is heated to 75°C. The pH of the solution is adjusted to pH 5 - 8, preferable at 6.5 by addition of nitric acid or hydrochloric acid. 119 g of a cobalt (II) nitrate solution (6% Cobalt content) are slowly added to the reactor. The pH-value is maintained via NaOH (32%). The suspension is equilibrated for 30 min. Then the pH-value is adapted to 8-10 by dosage of a sodium hydroxide solution (32%). 65.3 g of a Strontium chloride solution (11.1 % Strontium content) are added to precipitate $Sr(OH)_2$. The pH is maintained using sodium hydroxide (32%).

The suspension is cooled down and the solid remaining is decanted and washed to remove all side products. The pigment is transferred to a porcelain crucible and dried over night at 110°C. After sieving with a 0.3 mm sieve the powder is calcined at 880°C for 30 min. A night blue $SrCo_2(PO_4)_2$ pigment with lustre is obtained.
L value = 33, a = 13, b = -22
These values are measured using a Minolta colour unit CR-300. The Lab values can differ depending on the applied layer thickness and morphology.

Example 4: Eye Shadow

| Phase A | |
|---|---|
| 30% | Pigment of Example 1 |
| 7.5% | Potato starch |
| 49.5% | Talc |
| 2.5% | Magnesium stearate |
| Phase B: | |
| 9.14% | Isopropyl stearate |
| 0.53% | Cetyl palmitat |
| 0.53% | Ewalin 1751 (Petrolatum) |
| 0.2% | Fragrance Elegance 79228 D MF (Perfume) |
| 0.1% | Propyl-4-hydroxybenzoate (Propylparabene) |
| Total: | 100% |

The components of Phase A are mixed homogeneousty. Thereafter one adds the powder mixture with ongoing stirring to Phase B. The final powder will be pressed, using 40-50 bar pressure, afterwards.

Example 5: Nail varnish

2% Pigment of Example 2
98% Thixotrope nail varnish base 1348 (toluene, ethyl acetate, butyl acetate, nitrocellulose, tosylamide/formaldehyde resin, dibutyl phthalate, isopropyl alcohol, stearalkonium hectorite, camphor, acrylates copolymers, benzophenone).

All components are intensively mixed to get a homogeneous system.


**Claims**

1. Effect pigments based on substrates comprising at least one layer, which consists of a complex phosphate system.

2. Effect pigments according to claim 1, **characterized in that** the complex phosphate system has the formula $A_p B_q P_x O_y$ with

   A = ammonium, metals of group 1, 2, 11 and/or 12 of the periodic table
   B = elements of group 3 to 15 of the periodic table, lanthanides and/or actinides

   p = 1,2, ....r
   q=1,2,....s
   x=1,2....n
   y = 2,....m

3. Effect pigments according to claim 1 or 2, wherein the substrate is platelet-shaped, spherical or needle-shaped.

4. Effect pigments according to claim 3, wherein the substrate is platelet-shaped and is of mica, $SiO_2$, aluminium oxide, glass, micaceous iron oxide, oxidised graphite, aluminium oxide-coated graphite, basic lead carbonate, barium sulphate, chromium oxide, BN, MgO, magnesium fluoride, $Si_3N_4$, metal, effect pigments or effect multilayer pigments, or of coated or uncoated $SiO_2$-spheres or needle-shaped iron oxides.

5. Effect pigments according to claim 3, **characterised in that** the metal is aluminium or titanium.

6. Effect pigments according to one of claims 1 to 5, **characterised in that** the pigments are further coated on top of the complex phosphate system with one or more layers of metal oxides, metal oxide hydrates, metal fluorides and/or semitransparent metal layers.

7. Process for the preparation of a pigment according to claim 1, **characterised in that** a substrate is coated with at least one inorganic phosphate and in the case of coating with only one inorganic phosphate with at least one further metal component, followed by annealing to form the complex phosphate system.

8. Process according to claim 7, **characterised in that** the substrate is platelet-shaped and is of mica, $SiO_2$, aluminium oxide, glass, micaceous iron oxide, oxidised graphite, aluminium oxide-coated graphite, basic lead carbonate, barium sulphate, chromium oxide, BN, MgO, magnesium fluoride, $Si_3N_4$, metal, effect pigments or effect multilayer pigments, or of coated or un-coated $SiO_2$-spheres or needle-shaped iron oxides.

9. Use of a pigment according to one of claims 1 to 6 in paints, lacquers, printing inks, powder coatings, paper coatings, plastics, cosmetics, inks, glazes for ceramics and glasses, decorative applications for foods and drugs and security-enhancing features.

**European Patent Office**

**EUROPEAN SEARCH REPORT**

Application Number

EP 04 00 9622

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 423 131 B1 (ZICKGRAF REINHARD ET AL) 23 July 2002 (2002-07-23) | 1,2,4, 6-9 | C09C1/00 |
| A | * column 2, lines 4-57; example 4 * <br> * column 1, lines 12-14 * <br> ----- | 3,5 | |
| X | US 3 331 699 A (CUPERY MARTIN E ET AL) 18 July 1967 (1967-07-18) | 1-4,7-9 | |
| A | * column 1, lines 11-15; examples 10,17 * <br> ----- | 5,6 | |
| X | US 3 108 885 A (DUNSETH MARIA G) 29 October 1963 (1963-10-29) | 1,2,7,9 | |
| A | * claims 1,2; example 2; table 2 * <br> ----- | 3-6,8 | |

TECHNICAL FIELDS
SEARCHED (Int.Cl.7)

C09C

The present search report has been drawn up for all claims

| Place of search | Date of completion of the search | Examiner |
|---|---|---|
| Munich | 15 July 2004 | Werner, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 00 9622

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2004

| Patent document cited in search report | | Publication date | Patent family member(s) | | Publication date |
|---|---|---|---|---|---|
| US 6423131 | B1 | 16-05-2002 | DE | 19840156 A1 | 09-03-2000 |
| | | | DE | 59901172 D1 | 16-05-2002 |
| | | | EP | 0984044 A1 | 08-03-2000 |
| | | | ES | 2175884 T3 | 16-11-2002 |
| | | | JP | 2000086930 A | 28-03-2000 |
| | | | US | 2002056402 A1 | 16-05-2002 |
| US 3331699 | A | 18-07-1967 | NONE | | |
| US 3108885 | A | 29-10-1963 | NONE | | |

EPO FORM P0459

For more details about this annex : see Official Journal of the European Patent Office, No. 12/82